# EUROPEAN PATENT APPLICATION

(11) **EP 1 348 423 A1**
(43) Date of publication of application: **01.10.2003**
(21) Application number: 02006960.5
(22) Date of filing: 27.03.2002
(51) Int. Cl.: A61K 7/46, A61L 9/05

(54) **Fragrance compositions**

(71) Applicant: Givaudan SA, 1214 Vernier (CH)
(72) Inventor: Quellet, Christian, 2502 Biel (CH); Schudel, Markus, 8050 Zürich (CH)
(74) Representative: Simmons, John Murray, Dr.

(57) **Abstract**

The invention relates to microencapsulated fragrance compositions formed by spray drying an oil-in-water emulsion wherein the encapsulating material is a macromolcular surfactant, for example polyvinyl alcohol. The encapsulating material contains no additional surfactant material.

## Description

This invention is concerned with fragrance compositions intended for use in perfumed articles and devices, and in particular which can control the activation and diffusion of perfume in time in response to an external stimulus that is moisture.

The prior art has addressed such compositions and provides microcapsules wherein a fragrance composition is encapsulated in a polymeric matrix which protects said fragrance composition from its immediate environment and acts as means for the controlled diffusion of fragrance. A popular and convenient method of producing such encapsulated formulations consists of converting emulsions consisting of a dispersed oil phase and a continuous water phase, into solid form by removal of water, e.g. by spray drying. Compositions formed in this manner produce excellent results in terms of high loading of fragrance oil and good retention of the fragrance oil during processing and storage.

A particular example of such a formulation is disclosed in US 4,803,195 (Firmenich). There is disclosed a perfuming composition with deodorant or antiperspirant action which controls the activation and diffusion of fragrance over time when exposed to moisture, in particular sweat. The microcapsules are formed of a solid film-forming polymer, for example modified starch or polyvinyl alcohol, and an emulsifying agent. The emulsifying agents are chosen from octenyl succinte-substituted starches, mono- or diglycerides of fatty acids, esters derived from fatty acids and sorbitol or a saccharide, or their alkoxylated derivatives, or an ester of tartaric, citric, ascorbic, or lactic acid.

This document teaches that the particular combination of the film-forming polymer and the emulsifier permits of a process of "re-encapsulation". This "re-encapsulation" phenomenon permits of successive activations of perfume without multiple applications of composition to the skin. Activation occurs upon exposure of the composition to moisture.

US 5,508,259 also to Firmenich, discloses similar compositions. The compositions comprise film-forming polymer and emulsifier as hereinabove described. Similarly, it is the combination of film-forming polymer and emulsifying agent that permits of the so-called "re-encapsulation" phenomenon. Such a composition may be combined with other and distinct perfume elements in liquid form such that the liquid element can provide a continuous odour impulse, and the microencapsulated fragrance can give a different impulse upon activation.

Microencapsulated compositions can be produced with high perfume loading using, for example spray-drying techniques from oil-in-water emulsions. The prior art also shows that such compositions can be successively activated to diffuse fragrance upon exposure to moisture, provided one uses a film-forming polymer in conjuction with a low molecular weight emulsifier.

However, a problem with such prior art composition resides in the use of the emulsifying agents which are all relatively low molecular weight materials having molecular weights M_{w} considerably below 10,000 Daltons. These low molecular weight materials can desorb quite rapidly from the oil-water interface and may cause reversible or irreversible destabilisation of the dispersed oil phase, for example through coalescence, phase inversion, Ostwald ripening, flocculation, creaming or sedimentation. Still further, the applicant has found that the presence of certain emulsifying agents disclosed in the prior art references, in particular the modified starches, in perfumed articles caused those articles to discolour, e.g. to take on a greyish or brownish hue when exposed to high relative humidity or ambient product or body moisture. Naturally, this is unpleasant for end users. Further still, whereas prior art compositions activate to release fragrance before re-encapsulating under dry conditions, applicant has found that compositions containing low molecular weight emulsifying agents may be somewhat unstable in the presence of continuously moist or humid conditions, and may not resist rapid loss of large amounts of fragrance material.

Accordingly, there remains a need to provide fragrance compositions intended for use in perfumed articles and devices that permit of high loading of fragrance material and good retention during storage, and which will diffuse fragrance material on demand in a controlled and long-lasting manner, in response to a triggering stimulus, such as the presence of moisture, and which is not prone to discolouration and which can release fragrance material in a controlled manner even under conditions of prolonged high moisture levels.

Contrary to the teaching of the prior art, the applicant surprisingly now has found that it is possible to encapsulate fragrance material with high perfume loading, e.g. exceeding 50%, with excellent retention, and unexpectedly low amount of surface oil during processing and storage, and to form fragrance compositions having all the benefits described above without the need to use a low molecular weight emulsifying agent.

Therefore the invention provides in a first aspect a fragrance composition consisting essentially of particles of fragrance material encapsulated by a macromolecular surfactant.

The term "macromolecular surfactant" as used hereing refers to materials that are capable of forming structured interfacial films around droplets of fragrance material and thereby effectively prevent coalescence of the droplets and so are capable of stabilising the droplets. Accordingly, compositions of the present inventions do not need to employ additional low molecular weight emulsifiers.

Macromolecular surfactants useful in the present invention may be selected from those known water-soluble or water-swellable synthetic polymers known in the art as polymeric emulsifiers or colloid-stabilising polymers.

Examples of suitable macromolecular surfactants are:
(1) Homopolymers, e.g.
   - polyvinyl compounds, e.g. poly (vinyl acetate), poly (vinyl alcohol) or poly (vinyl pyrrolidon);
   - polycarboxylic acids, e.g. poly(acrylic acid) and poly(methacrylic acid);
   - polysulfonic acids, e.g., poly (styrene sulfonic acid);
   - polyesters, e.g. glycol polyacrylate;
   - polyamides, e.g. poly (acryl amide);
   - polyurethanes, such as polyurethane that contains ionic groups, e.g. carboxylic acid, sulfonic acid or tertiary amines; or such as polyurethane that contains nonionic hydrophilic groups, e.g. ethylene oxide;
   - poly (ethylene oxide), poly(propylene oxide) and further polyalkylene glycol derivatives;
(2) polycondensates, for example
   - ethoxylated phenol - formaldehyde resins
   - sulfonated aromatic formaldehyde resins
   - urea or melamine - formaldehyde compounds
   - polyamide, polyamine, and epichlorohydrin resins
(3) AB copolymers (wherein A is a more water-soluble or water swellable moiety and B is a less water-soluble or water-swellable moiety) selected from
   - styrene copolymers, e.g. styrene-acrylic acid polymers or styrene-ethylene oxide polymers
   - copolymers of polyvinyl and maleic acid compounds, e.g. styrene - maleic anhydride polymers or vinyl acetate-maleic acid ester polymers
   - polyvinyl - polyalkylene copolymers, e.g. vinyl acetate
   - ethylene polymers, ethylene - acrylic acid - acrylic acid ester polymers or ethylene - acrylic acid-acrylonitrile polymers
   - other vinyl copolymers, e.g. vinyl acetate polymers, acrylic acid - acrylonitrile polymers, acrylic acid-acryl amide polymers;
(4) ABA block copolymers selected from
   - for "A" water-soluble or water-swellable moieties such as poly(ethylene oxide), poly (vinyl alcohol), poly (acrylic amid), poly (acrylic acid), poly (vinyl pyrrolidon), or poly(caprolactone);
   - for "B" less water-soluble or sparingly water-soluble moieties such as poly (propylene oxide), poly (vinyl acetate), poly (vinyl butyral), poly (lauryl methacrylate), polystyrene , poly (hydroxystearic acid), polysiloxane,
(5) B(A)ₙ graft or comb polymers selected from
   - for "A" from water-soluble or water-swellable moieties such as vinyl alcohol, vinyl acetate, ethylene oxide, propylene oxide, vinyl sulphonates, acrylic acids and vinyl amines,
   - "B" is selected from vinyl polymer chains or siloxane chains,
(6) Natural polymeric emulsifiers such as cellulose derivatives, e.g. carboxymethylcellulose, hydroxypropylmethylcellulose, methylcellulose and other derivatives thereof,

In addition, plasticisers may employed to modify the manner in which the macromolecular surfactant releases fragrance material. Suitable plasticisers such as triacetin, triethyl citrate, polyethylene glycol, diethyl phthalate, tributyl citrate, glycerine or other conventional plasticizers and their mixtures.

Macromolecular surfactants are of high molecular weight, in particular they are materials having molecular weights M_{w} of 10000 Daltons or more.

In a preferred embodiment the macromolecular surfactant is a polyvinyl alcohol, for example those selected from commercially available products such as Mowiol® (ex Clariant SA, Switzerland). In particular, those PVAs having a degree of hydrolysis of between 70 and 100%, more particularly between 80 and 90%, with the highest emulsifying power being met at 88% hydrolysis.

Preferred polyvinyl alcohols have a viscosity measured at room temperature and as a 30%(wt) solution in water at a shear rate of 100s⁻¹ lower than about 10,000 mPas, more preferably below about 5,000 mPas. Polymers having viscosities above this level become increasingly difficult to use in standard commercially available spray driers, granulators or other manufacturing equipment. In order to ensure that the polymer has viscosity in the desired range, one preferably employs a polyvinyl alcohol having a molecular weight M_{w} of above 10,000 Daltons but lower than about 40,000 Daltons.

In a preferred embodiment the viscosity of polyvinyl alcohol is further reduced by oxidative breakdown of the polymer chains. In particular, residual 1,2-glycol units present in the native polymer are cleaved oxidatively. This process may be carried out under mild conditions in a manner known per se, for example using sodium periodate at a level of about 1 to 5%(wt) in a 20 to 30% solution in water at room temperature. The 1,2-glycol cleavage with sodium periodate takes place virtually spontaneously even at room temperature. Breakdown of the polymer chains with other oxidising agents, e.g. peroxides could also be used but mostly under harsher conditions. The quantities of peroxide needed to achieve a given viscosity and the reaction conditions have to be determined individually for each grade of polyvinyl alcohol as further described in the publically available literature for Mowiol®, (Clariant AG, Switzerland).

The macromolecular surfactant may be present in amounts of 20 to 99%(wt) of the total fragrance composition, and it may be used individually or as mixtures of two or more materials.

Fragrance materials for use in compositions of the present invention may be selected from natural products such as essential oils, absolutes, resinoids, resins, concretes, and synthetic perfume components such as hydrocarbons, alcohols, aldehydes, ketones, ethers, acids, acetals, ketals and nitriles, including saturated and unsaturated compounds, aliphatic, carbocyclic and heterocyclic compounds, or precursors of any of the above. Other examples of odorant compositions which may be used are described in H 1468 (United States Statutory Invention Registration).

The amount of perfume possible to be incorporated into the fragrance composition may be up to 50 wt%, in some cases even up to 80 wt%, e.g. 1 to 80% based on dry material, with a high perfume retention close to 100% of even the very volatile components having a Loss Factor of greater than 10² Pa ppm. The term « Loss Factor » refers to a parameter that is related to the losses of fragrance material during drying and is defined as the product of the pure component vapour pressure (Pa) and the water solubility (ppm) at room temperature. Vapour pressures and water solubility data for commercially available fragrance components are well known and so the Loss Factor for a given fragrance component may be easily calculated. Alternatively, vapour pressure and water solubility measurements may be easily taken using techniques well known in the art. Vapour pressure of fragrance components may be measured using any of the known quantitative headspace analysis techniques, see for example Mueller and Lamparsky in Perfumes: Art, Science and Technology, Chapter 6 "The Measurement of Odors" at pages 176 - 179 (Elsevier 1991). The water solubility of fragrances may be measured according to techniques known in the art for the measurement of sparingly water-soluble materials. A preferred technique involves the formation of a saturated solution of a fragrance component in water. A tube with a dialysed membrane is placed in the solution such that after equilibration an idealised solution is formed within the tube. The tube may be removed and the water solution therein extracted with a suitable organic solvent to remove the fragrance component. Finally the extracted fragrance component may be concentrated and measured, for example using gas chromatography. Other methods of measuring fragrances are disclosed in Gygax et al, Chimia 55 (2001) 401-405.

Compositions according to the present invention may contain other optional excipients, for example anti-static agents, flowing agents, and other agents commonly known in the art. Excipients may be present in amounts of up to about 1% (wt) of the total composition.

The fragrance compositions of the present invention may be provided in multi-particulate form, for example as a spraydried powder. Preferably, the particle size is between 0.01 and 2 mm. However, further processing of compositions is contemplated. For example, the compositions may be granulated in order to increase the particle size of the formulation which may be required for certain applications, e.g. when the formulation is to be mixed with a coarse material. Alternatively, the composition of the present invention may be dispersed in a matrix material, e.g. a hydrophilic glassy matrix material according to an extrusion process.

Fragrance compositions according to the present invention are suited for incorporation into consumer products, for example personal and household care products, and in particular said products which, in use, come into contact with moisture, e.g. deodorants, soaps and detergents, or diapers, humidity absorbers, air-fresheners and related air-purification products.

In a particularly preferred embodiment the fragrance composition may be admixed with a granular humidity adsorbing agent such as CaCl₂ and the like, in an air purification and room deodorising device as well as for the use in households where ever moisture occurs accompanied by malodour, e.g. in closets, wardrobes, shoes and waste bins.

The amount of fragrance composition employed in perfumed products or articles according to the present invention may vary according to the particular application in which it is employed and on the fragrance loading in the fragrance composition. For deodorant applications, one may employ fragrance composition in amounts from 0.05 to 20 by weight of fragrance material based on the total weight of the deodorant.

In another aspect of the invention there is provided a process of forming a fragrance composition comprising the step of dispersing or emulsifying an oil phase containing fragrance material in a continuous phase consisting essentially of water and a polymer matrix material as hereinabove defined, and thereafter removing water, to provide the fragrance composition in particulate form.

Water may be removed in a manner known *per se.* For example, the emulsion or dispersion may be spray dried, spray-chilled, granulated, agglomerated or extruded according to methods known per se. Preferred manufacturing methods are described in more detail in the Example.

In order to ensure relatively quick processing times and thereby to ensure high encapsulation efficiency and good retention of fragrance oil, the amount of water to be eliminated by the drying step should be as low as possible, for example lower than 80%.

The fragrance composition of the present invention may be in the form of liquid droplets of fragrance oil dispersed in a solid polymer matrix of macromolecular surfactant. The mass fraction of liquid fragrance oil to solid polymer matrix may be measured by standard pulsed NMR techniques, or magnetic imaging techniques as further described in E. Dickinson, Advances In Food Colloids, Blackie Academic and Professional, 1996 at pages 145 through 175.

There now follows a series of Examples that serve to illustrate embodiments of the present invention. It will be understood that these Examples are illustrative, and the invention is not to be considered as restricted thereto except as indicated in the appended claims.

### Example 1:

Preparation of encapsulates according to the invention: 10.0 kg of polyvinyl alcohol Mowiol® 4-88, (Clariant AG, Switzerland) were dissolved in 90.0 kg deionised hot water (70°C) to result in 10% polyvinyl alcohol solution. After cooling down to ambient room temperature 11.3 kg of a typical air freshener perfume (Givaudan Vernier SA, Switzerland) were added and homogenised using a Ultra-Turrax T-52 (IKA GmbH,Germany) at maximum speed for 5 minutes. The resulting emulsion had a water content of ca 80 wt% and a perfume droplet size of 0.8 µm measured with Olympus BX50 light microscope (Olympus, Japan). The dynamic viscosity of the emulsion was 47 mPa·s (shear rate: 100 s⁻¹) measured with a Modular Compact Rheometer MCR 300 (Physica, Germany).

This emulsion was further spray dried using the pilot plant from Nubilosa (Nubilosa Molekularzerstäubung GmbH, Germany) with a water evaporation capacity of 33 kg/h (air throughput of 1'500 m³/h) at 150°C inlet and 70°C outlet temperature and further equipped with a two fluid nozzle operated at 3.0 bar air pressure.

The resultant powder had a total oil content of 51.2 wt% (0.6 wt% surface oil content) measured by standard HPLC procedures compared to a theoretical payload of 53.0 wt% perfume resulting in a perfume oil recovery (initial retention) of 97% related to the HPLC total oil measurements. The total oil content measured by pulsed NMR method using an Oxford MQA6005 (Oxford Instruments IAG, UK) resulted in 53 wt% total oil content (corrected for a residual moisture content of 3 wt% measured by Karl-Fischer) and reflecting that close to 100 wt% of the perfume is present as liquid droplets dispersed in a polymer matrix.

### Example 2:

### Preparation of a comparative sample (Sample 1):

The sample 1 was prepared by a similar procedure to that described above for the sample of the present invention whereby the matrix material and the emulsifier where dissolved in water prior to homogenising it with the same air freshener perfume used for the sample of the presnt invention. Furthermore, for Sample 1 a Niro Mobile Minor (Niro A/S, Denmark) was used with a rotary atomiser and an inlet temperature 170°C and an outlet temperature 80...84°C.

**Table 1:**

| Preparation of Sample 1 encapsulate | | | | |
|---|---|---|---|---|
| Sample | Matrix Material maltodextrin DE=6 [g] | Emulsifier Capsul®[g] | Perfume [g] | Water [g] |
| 1 | 693 | 189 | 490 | 1280 |

| | | | | |
|---|---|---|---|---|
| - Capsul® : mod. starch emulsifier (National Starch and Chemicals Ltd., UK) | | | | |
| - Perfume: same air freshener perfume as used above | | | | |
| - Sample 1 based on US 4,803,195 (Firmenich) | | | | |

**Table 2:**

| Properties of Sample 1 encapsulate. | | | | |
|---|---|---|---|---|
| Sample | Total oil NMR [wt%] | Theor. Payload [wt%] | Initial Retention [%] | Residual moisture content [wt%] |
| 1 | 30 | 36 | 83 | 4 |

### Example 3:

### Application tests:

### a) Storage test:

20.0 g of standard CaCl₂ granulates used for humidity absorber products containing the encapsulates (sample of the present invention and Sample 1) on a 0.4 wt% perfume level and a blank containing no perfume or encapsulates were placed in 100 ml bottles and 10.0 g of deionised H₂O were added. The open bottles were placed in a climate chamber for 3 days at 37°C and 70% humidity. After 3 days the coloration were inspected visually (see table 3).

**Table 3:**

| Visual evaluation of storage test. | | | |
|---|---|---|---|
| Capsule | Total oil [wt%] | Coloration | Preferred |
| **sample(invention)** | **53** | **white** | **1**^{**st**} |
| CaCl₂ blank | - | white | 1^{st} |
| Sample 1 | 30 | yellow-orange | worst |

### b) Perfume release test:

20g of CaCl₂ granulates mixed with encapsulates on a perfume level of 0.4 wt% were placed in an open box in a closed climate room of ca. 2 m³ at 25°C and 60...70% relative humidity. After 7 days the air in the room were olfactory evaluated by experts whereas the following results were found given in table 4:

**Table 4:**

| Olfactory evaluation of application test. | | | | |
|---|---|---|---|---|
| Capsule | Payload [wt%] | Intensity | Quality | Preferred |
| **sample** | **53** | **very strong** | **intact perfume** | **1**^{**st**} |
| **(invention)** | | | | |
| Sample 1 | 31 | strong | intact perfume | 2^{nd} |

In addition, the inventive sample was described by perfumers as "fresh" whereas the Sample 1 was referred to as "humid".

## Claims

1. A fragrance composition comprising particles of fragrance material consisting essentially of particles of fragrance material encapsulated by a macromolecular surfactant

2. A fragrance composition according to claim 1 wherein the macromolecular surfactant is polyvinyl alcohol having a molecular weight of more than 10,000 Daltons and less than 40,000 Daltons.

3. A fragrance composition according to claim 2 wherein the polyvinyl alcohol has a degree of hydrolysis of between 70 and 100%.

4. A perfumed article containing a fragrance composition as defined in any of the preceding claims.

5. A perfumed article according to claim 4 wherein it is a deodorant composition.

6. A perfumed article according to claim 4 wherein it is a humidity absorber.

7. A perfumed article according to claim 6 comprising Calcium chloride.

8. A perfumed article according to claim 4 wherein it is a soap.

9. A fragrance composition according to any of the claims 1 through 3 in particulate form.

10. A process of encapsulating a fragrance material comprising the step of dispersing or emulsifying an oil phase containing fragrance material in a continuous phase consisting essentially of water and a macromolecular surfactant as defined above, and thereafter removing water, to provide the fragrance composition.
